# EUROPEAN PATENT APPLICATION

(11) **EP 2 362 189 A2**
(43) Date of publication of application: **31.08.2011**
(21) Application number: 10002725.9
(22) Date of filing: 16.03.2010
(51) Int. Cl.: G01D 5/14

(54) **Magnetic triangulation based position sensor**

(30) Priority: 27.01.2010 ES 201000095
(71) Applicant: Inescop Instituto Tecnologico de calzado y conexas, 03600 Elda Alicante (ES)
(72) Inventor: The designation of the inventor has not yet been filed
(74) Representative: Fernandez Lerroux, Aurelio

(57) **Abstract**

The present invention refers to a magnetic triangulation based position sensor which can be used for determining the relative position of an object or body part, in particular one that is completely or partially hidden, with respect to the object that hides it, which is based on a set comprising one or more magnets (1) which are fixed on to the object to be analysed (5) (foot, head, hand, torso, or any other object), and three or more magnetic field sensors (2), which are placed on a sensor support (3) that is then fixed onto the object so that when within the range of the magnetic field (8) of the magnet (1), the sensors will generate a signal proportional to the magnetic field present in that area, so that for each relative position of the magnet (1) with respect to the sensor support (3), the sensors (2) will generate a characteristic signal corresponding to that position. In this way, by applying a suitable mathematical algorithm it will be possible to establish the relationship between the relative position of the magnet (1) with respect to the sensor support (3) and hence the relative position of the object or body part (5) with respect to another object (4).

## Description

### OBJECT OF THE INVENTION

The present description refers to a patent for the invention of a magnetic triangulation based position sensor intended for the determination of the relative position of an object, a part of the human body or an animal, especially those parts that are hidden or only partially visible. This would be achieved via sensors which detect the magnetic fields generated by a magnet or a set of magnets which are placed on the object in such a way that the magnetic field lines go through the material or materials that hide the object and prevent the positioning of a part with respect to a given point to be known.

### FIELD OF THE INVENTION

This invention could be used when it is necessary to ascertain the relative position of a human body part when inside another object or when completely or partially covered with material, making it impossible to ascertain its position. It could be especially useful in the footwear, head wear, glove and clothing production research sector as well as for medical or sports purposes. It could also be applied to ascertain the relative position of whatever hidden or partially or completely covered object or the relative position of a part of an animal's body.

### BACKGROUND OF THE INVENTION

Currently determining the position of an object or part of the human body when inside or partially covered by another object, for example a shoe, glove, helmet, etc. is only possible by using X-rays and/ or magnetic resonance techniques.

Although being useful in some contexts, these techniques have certain drawbacks as they have to be used in very specific conditions which also results in an elevated cost of the procedure.

As well as the known disadvantages of radiation and the need to work in controlled environments, X-ray and magnetic resonance techniques use large machines and cannot often capture dynamic patterns. Furthermore, in specific cases when this is in fact possible the process is very difficult to achieve and in any case, the procedure can be quite long or in everyday conditions for the person or animal being analysed.

The development of everyday items such as shoes, helmets, gloves, etc. is advancing in the lines of providing the best possible ergonomic features which makes it essential to analyse the behaviour of the part of the human body placed inside said object. This is dynamic behaviour and involves constant position changes, as the object may be comfortable in one position and not in another. X-ray and magnetic resonance techniques do not permit such dynamic behaviour to be recorded.

It is this necessity to locate the relative position of the body parts when inside other objects, for example a foot in a shoe, a hand in a glove, a head in a helmet which has led to the development of this position sensor. This sensor is not only applicable for detecting the position of part of the body when hidden or partially covered by another object, but also for ascertaining the relative position of any object that is partially or completely covered or hidden, and obviously, of parts that are visible too.

The above described need is demonstrated clearly in the footwear industry; if the position and movements of a foot can be ascertained whilst inside a shoe, footwear could be designed that fits and could adapt to the needs of the foot. It is important to note that it could be used in other fields as well however, such as ascertaining the behaviour of motorcyclists and skiers when determining the effectiveness of helmets in accidents. It is also useful in ascertaining animals' comfort levels, in that due to lack of speech, triangulation sensors could be the only way of analysing a multitude of aspects, such as the effects of a horse saddle or tread impacts on joints.

Different position sensors described in numerous patents exist, including magnetic field sensors, but the applicant is not aware of any patents related to systems, machines or devices which leads to detecting or verifying the relative position of a hidden or partially visible object or part of an object using magnetic field sensors apart from the above-mentioned X-ray and magnetic resonance techniques. No system or device is known that allows the position of the object to be dynamically monitored or controlled.

The signals provided by the magnetic field sensors will depend on the relative position of the sensors with respect to one another, their sensitivity and the physical shape of the magnet, as well as the intrinsic features of the magnet, such as the chemical composition and magnetic potential that it can provide. This makes it especially difficult to ascertain the univocal relationship between the relative position of the sensors and magnet and the magnetic footprint. In all the patents analysed no linearization through the use of a correspondence table between the electrical signals proportional to the magnetic field and the relative position of the magnet or magnets with respect to the sensors has been described. This also represents an important novelty of this invention in that an univocal relationship is established between the magnetic footprint captured by the sensors and the position of the magnet, that is to say: for each magnet position there is a specific magnetic footprint defined by the signals of the magnetic field.

### DETAILED DESCRIPTION OF THE INVENTION

The magnetic triangulation based position sensor which is the object of the present invention, applicable for determining the relative position of an object, or part of the human body or animal with respect to another object which partially or completely covers or hides it, is based on a magnet or a set of magnets (1) that are attached to the part to be analysed (5), namely foot, head, hand, torso or any other object, and preferably three or more magnetic field sensors (2), which are assembled on a support (3) that is fixed on to the object (4) in such a way that it is within the magnets' (1) magnetic field radius (8), thus generating a signal proportional to the magnetic field (8) present In that area. This way, for each relative position of the magnet with respect to the sensor support (3), the sensors (3) will generate a characteristic signal that corresponds with that position, hence by applying an adequate mathematical algorithm it will be possible to establish a relationship between the relative position of the magnet (1) with respect to the sensor support (3).

The magnetic field sensors (2) can be placed on the same plane (H1A, H1B, and H1C) or on different planes (H2A, H2B, and H2C),

Therefore the position sensor is comprised of the following elements:
- Magnet (1) manufactured with any common technology, which is located in the area or point whose position is to be ascertained. This can be made up of one or more magnets.
- Magnetic field sensors (2), which provide an electrical signal which is proportional to the magnetic field (8) present in the area in which they are placed. They can feature different technologies and be preferably three or more sensors, but the number Is variable and in certain cases one could suffice.
- Magnetic sensor support (3) where the sensors are held (2) at certain distances from one another.
- Electronic control (6), which is in charge of managing, conditioning, filtering, and A/D converting the signals provided by the magnetic field sensors, providing the signals in a suitable format for the processing unit (7).
- Processing unit (7), which is made up of a set of electronic circuits that process the signals of the electronic control and provide a signal detailing the position of the magnet (1) with respect to the sensor support (2).

Fixing the magnet or magnets (1) to the body part (5) or the object whose relative position is to be ascertained can be done using any technique whether it may be directly onto the object (5), or using covers equipped with the corresponding magnets (1). It is important that the magnet cannot be moved or displaced (1) with respect to the object (5) and that the object and magnets move together.

Moving the body part or object (5) should produce the same movement from the magnets (1), with respect to the object which covers it (4) and the magnetic field sensors (2) arranged on the same, in such a way that moving the magnet or magnets (1) would change the magnetic field (8), and alterations of the same with respect to the initial position, are captured by the sensors (2). This would lead to ascertaining the relative position of the object or body part (5) with respect to the object which hides it (4) and a dynamic environment, that is to say, relative position whilst moving.

The control unit (6) manages the signals received from the sensors (2), and the processing unit (7) processes this signal and the information about the magnets' position (1) in relation to the sensor support (3) and translates such position into a graphic visualisation of the hidden object (5) with respect to the object or body that hides it (4).

The signals between the sensors (2) and the control unit (6) and between the control unit and the processing unit (7) are transmitted using any known wireless or non-wireless method.

In order to determine the position from the electrical signals previously provided by the magnetic field sensors the correspondence between each position of the magnet in relation to that of the magnetic field sensors in the useful work volume of the sensor is established. To do this, a correspondence table will be produced in which the magnet's position and the value of the electrical signal from the magnetic field sensors in each position can be compared. In this way the exact position can be determined from the electronic signals provided by the magnetic field sensors.

### BRIEF DESCRIPTION OF THE DRAWINGS

To better understand the claimed object, it will be represented by four sets of planes in which the following have been represented in an illustrative and not constraining manner:
**Figure number 1** is a schematic view of the set comprising the magnet (1) and the position sensor with magnetic field sensors (2) H1A, W1B and H1C arranged on the same planes and sensors H2A, H2B and H2C on different planes
**Figure number 2** is a schematic view of the magnet (1) and the sensors (2) in the sensor support (3), as well as a schematic representation of the magnetic field lines (8) that pass through the sensors.
**Figure number 3** represents a block diagram which shows the sensors (2) in the sensor support (3), the electronic control (6) and the calculation and signal processing unit (7).
**Figure number 4** is a schematic representation of the preferred embodiment of the invention showing the position of a foot (5) in a shoe (4), the sensors (2) in the sensor support (3), fixed to the shoe (4) and the magnet (1) fixed to the part (5) whose relative position with respect to the object (4) is to be ascertained.

### PREFERRED EMBODIMENT OF THE INVENTION

### EXAMPLE 1

**Figure number 4** illustrates a preferred embodiment of the invention. It is intended to ascertain the position and movement of a foot (5) within a shoe (4).

To do this the sensor support (3) which holds six magnetic field sensors (2), three on the same plane and 3 on a different plane is fixed onto a shoe (4) and the magnet (1) is attached to the foot (5) of which the relative position in relation to the object (4) is to be determined.

The magnet (1) is fixed with an adhesive to the foot (5) or by any other means, for example a sock equipped with a magnet (1) which prevents the magnet from moving (1) with respect to the foot (5); the sensor support (3) is placed within the range of the magnet (1), and the electronic control (4) provides the necessary electrical signals for the processing unit (5) to calculate the relative position of the sensor or sensors (2) with respect to the magnet (1).

Once the sensor support (3) has been placed within the range of the magnetic field (8) of the magnet (1), a signal will be generated proportional to the magnetic field (8) which is present in the area so that for each relative position of the magnet in relation to the sensor support (3) the sensors (3) will generate a characteristic signal corresponding to each position, and by applying the suitable mathematical algorithm the relation between the relative position of the magnet (1) with respect to the sensor support (3) can be established.

The sensors (2) transmit this information to the control unit (6) wirelessly, and then from there to the processing unit (7) -again, wirelessly.

The processing unit (7) translates the relative position of the magnet (1) with respect to the sensor support (3) providing a visualisation of the foot's position (5) with respect to the shoe (4).

This is a continuous process and lasts the amount of time that was predefined, either in the laboratory, using a treadmill, in which case wireless means for signal transmission are not necessary, or in an open field, which can be carried out due to the use of wireless technology.

This leads to the generation of information regarding the position of the foot (5) with respect to the shoe (4) in a dynamic environment, that is to say standing (5) and moving, in such a way that the relative position of the foot (5) with regards to the shoe (4) in many diverse conditions and positions of the foot is collected. This would be very valuable information to develop shoes which adapt perfectly to the foot in specific conditions, or which would allow better use of footwear for ergonomic or medical purposes by designing corrective, therapeutic, sports or any other footwear that is adapted for each activity.

### EXAMPLE 2

Another preferred embodiment of this invention would consist of detecting the position and movement of the head inside a helmet.

To do this, the sensor support (3), equipped with corresponding magnetic field sensors (2) would be fixed onto the helmet (4) and the magnet (1) would be placed on the head (5) of which the relative position inside the helmet (4) is to be ascertained.

The magnet (1) is fixed onto the head (5) using adhesives; the sensor support (3) is placed within the range of the magnet (1), and the electronic control (4) provides the necessary electrical signals for the processing unit (5) to calculate the relative position of the sensors (2) with respect to the magnet (1).

Once the sensor support (3) has been fixed into place within the magnetic field range (8) of the magnet (1), a signal will be generated which is proportional to the magnetic field (8) which is present in the area so that for each relative position of the magnet in relation to the sensor support (3), the sensors (3) will generate a characteristic signal which corresponds to that position. By then applying a suitable mathematical algorithm it will be possible to establish a relation between the relative position of the magnet (1) in relation to the sensor support (3).

The sensors (2) transmit this information to the control unit (6) wirelessly, and then from there to the processing unit (7) - again, wirelessly.

The processor (7) translates the relative position of the magnet (1) in relation to the sensor support (3) allowing a visualisation of the position of the head (5) in relation to the helmet (4).

This procedure is carried out in a continuous circuit using wireless technology.

This leads to information about the position of the head (5) with respect to the helmet (4) in a dynamic environment, that is to say: a driver being on a moving vehicle and therefore producing movement in the head (5) with respect to the helmet (4), which would make it possible to know the relative position of the head (5) with respect to the helmet (4) in very diverse driving conditions (acceleration, braking, in curves, etc.), which is very valuable information to develop safer helmets for the driver.

Likewise, the information can be analysed in test beds, simulating accidents to determine the effects of the impact and the helmet's performance.

With the position sensor proposed in this invention, different types of footwear, gloves, helmets, saddles, rackets and in general the relative position of whatever object which can at times be completely or partially covered by another object can be compared, for example: glove/hand, head/helmet, plaster cast/arm, observing the real interaction between the objects from the results obtained.

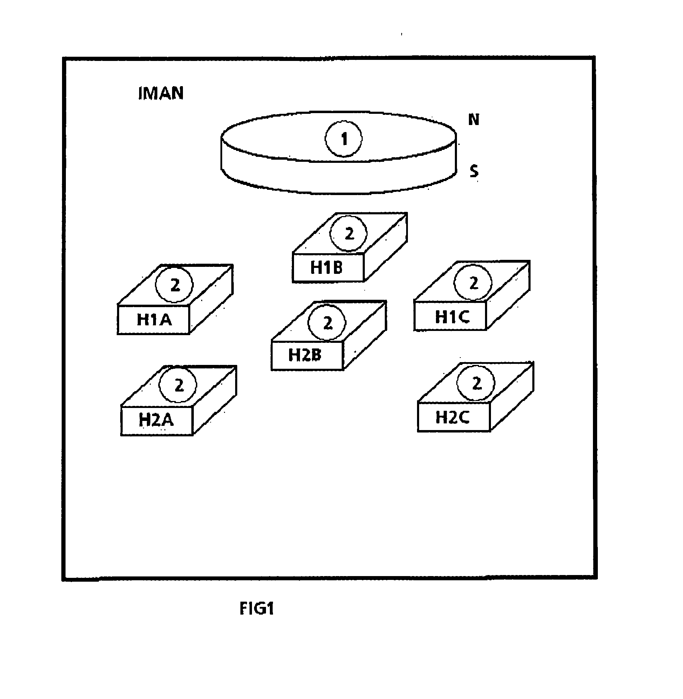

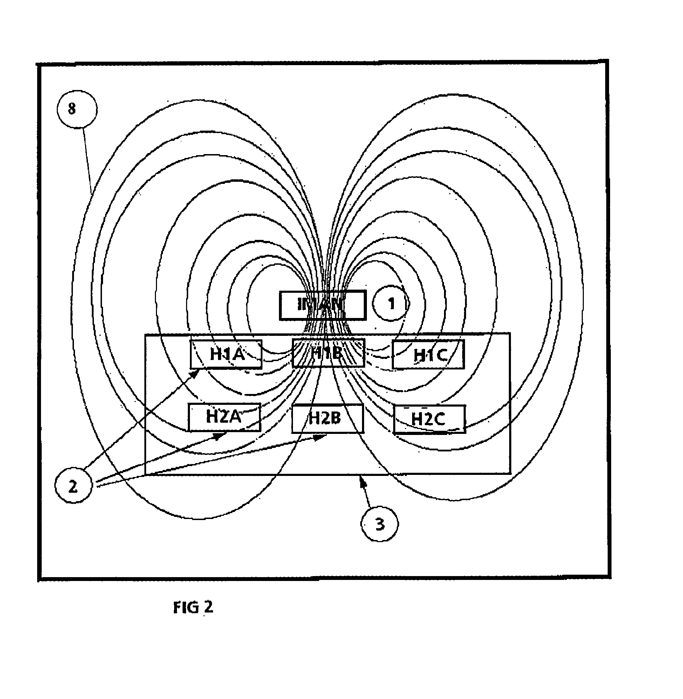

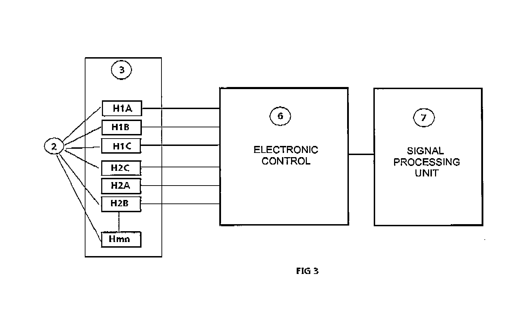

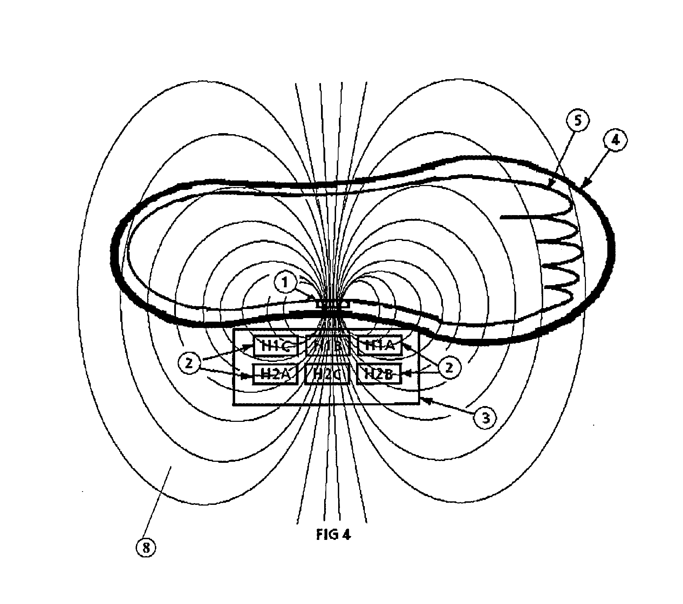

## Claims

1. Magnetic triangulation based position sensor applicable for determining the relative position of an object or a body part that is hidden or partly or completely covered by another object or material, using sensors which detect the magnetic field generated by a magnet or a set of magnets which are attached to the object in question, **characterised by** comprising one or more magnets (1); at least three or more magnetic field sensors (2); a sensor support (3); an electronic control unit (6) a processing unit (7) that measures the level of magnetic fields (8) with each sensor (2) and, by applying the adequate mathematical algorithm, can determine the relative position of the magnet (1) placed on the object or part (5) with respect to the object (4) on to which the sensor support (3) has been attached.

2. Magnetic triangulation based position sensor, according to the first claim, which is **characterised by** it establishing a univocal correspondence table between the relative position of the sensor and the magnets, and the electrical signals proportional to the detected magnetic field that they provide, defining each position from the linked magnetic footprint. From such a table the relative position can be established by comparing the magnetic signals detected by the magnetic field sensors and the relative position of the magnets.

3. Magnetic triangulation based position sensor: according to the first claim, which is **characterised by** the magnet or magnets (1) being fixed to the object (5) whose relative position with respect to the other object (4) is to be ascertained using whatever fixing technique that ensures that the magnet (1) and the object (5) move together as one.

4. Magnetic triangulation based position sensor, according to the first claim, which is **characterised by** the magnetic field sensor or sensors (2) being placed on the sensor support (3) which is fixed on the object (4) with respect to which the relative position of another object (5) is to be ascertained, whether it be on the same plane H1A, H1B and H1C or on different planes H2A, H2B and H2C. The sensors may be equipped with data transmission means based on either wireless or non-wireless systems, using radiofrequency or optoelectronics systems.

5. Magnetic triangulation based position sensor; according to the first claim, which is **characterised by** the electronic control unit (6) managing the signals provided by the magnetic field sensors (2), processing, filtering, and AID converting them, and then giving the signals the correct format for the processing unit (7). The sensors may be equipped with data transmission means based on either wireless or non-wireless systems, using radiofrequency or optoelectronics systems.

6. Magnetic triangulation based position sensor, according to the first claim, which is **characterised by** its processing unit (7) comprising a set of electronic circuits which processes the electronic control signals and provides a signal showing the position of the magnet (1) with respect to the sensor support (3) and translates the position into a graphic visualisation of the position of the hidden object (5) with respect to the object or body that hides it (4).

7. Magnetic triangulation based position sensor, according to the first claim, which is **characterised by** the magnetic field sensor or sensors (2) being uniaxial or multiaxial and being sensitive to magnetic fields in one direction or in multiple directions. The magnetic field sensors can be based on whatever technology that is electromagnetic, magnetoresistive or inductive, etc.
